# EUROPÄISCHE PATENTSCHRIFT

(11) **EP 1 163 882 B1**
(45) Veröffentlichungstag und Bekanntmachung des Hinweises auf die Patenterteilung: **24.09.2003**
(21) Anmeldenummer: 01113409.5
(22) Anmeldetag: 01.06.2001
(51) Int. Cl.: A61B 17/22

(54) **Vorrichtung zum Entfernen von Körpersteinen mit einem intrakorporalen Lithotripter**
Intracorporeal lithotripter for removal of calculi
Lithotripteur intracorporel pour l'enlèvement de calculs

(30) Priorität: 15.06.2000 DE 10029580
(43) Veröffentlichungstag der Anmeldung: 19.12.2001
(73) Patentinhaber: Ferton Holding SA, 2800 Delémont (CH)
(72) Erfinder: Hirt, Joachim, 78465 Konstanz (DE); Merkle, Wolfgang, 88709 Meersburg (DE)
(74) Vertreter: Gauger, Hans-Peter, Dipl.-Ing.

(56) Entgegenhaltungen:
- EP-A- 0 317 507
- EP-A- 0 421 285
- DE-A- 2 032 501
- DE-A- 19 609 019
- US-A- 4 178 935
- US-A- 5 911 699

## Beschreibung

Die Erfindung bezieht sich auf eine Vorrichtung zum Entfernen von Körpersteinen mit einem intrakorporalen Lithotripter gemäß dem Oberbegriff des Anspruches 1.

Für die Entfernung von Körpersteinen aus Körperhöhlen ist es bei Überschreitung einer für einen natürlichen Abgang noch ausreichenden Steingröße regelmäßig erforderlich, die Körpersteine zunächst zu zerkleinern, wobei die Zerkleinerung in kleine, spontan abgangsfähige oder direkt aus dem Körper ausspülbare Partikel vorgenommen wird. Die Zerkleinerung der Körpersteine wird dabei durch mechanische Druck- und Zugspannungen vorgenommen, die bei der intrakorporalen Lithotripsie mit dem distalen Ende einer als Wellenleiter dienenden Metallsonde auf die Körpersteine ausgeübt werden. Solche Spannungen führen zu einem Absprengen von Fragmenten aus der Oberfläche eines Steines und bewirken schließlich dessen Zertrümmerung. Für diese Steinzertrümmerung existiert allgemein das Problem einer geeigneten Energieübertragung mit besonderer Beachtung einer Vermeidung von Nebeneffekten auf das biologische Gewebe, das für die Steinzertrümmerung daher nicht als ein Widerlager dienen sollte.

Zur Durchführung einer intrakorporalen Lithotripsie ist aus der EP 0 421 285 B1 eine Vorrichtung gemäß dem Oberbegriff der ersten Anspruchs bekannt, bei welcher eine Metallsonde oder Sonotrode durch einen elektrisch angesteuerten Ultraschallwandler zu longitudinalen Schwingungen angeregt wird. Mit dem distalen Ende der in den Arbeitskanal eines Endoskops eingesetzten Sonde kann daher die Zertrümmerung eines Körpersteins veranlaßt werden. Der Ultraschallwandler ist dabei mit zwei piezokeramischen Scheiben ausgebildet, die zwischen einem Reflektor und einem Horn verspannt sind. Die beiden Scheiben werden für eine periodische Schwingungsanregung der Sonotrode durch eine Schaltungsanordnung angesteuert, die aus einem spannungsgesteuerten Oszillator besteht, dessen Ausgangssignal über einen Ausgangsverstärker und einen Ausgangsübertrager an die beiden piezokeramischen Scheiben angeliefert wird. Die Schaltungsanordnung umfaßt daneben noch einen Phasenkomparator, der die Phasen der Ausgangsspannung und des Ausgangsstroms des Ausgangsübertragers vergleicht sowie eine Regelspannung zur Steuerung des Oszillators erzeugt. Mit einer Vorrichtung dieser Ausbildung lassen sich die Körpersteine in aller Regel zu sehr feinen Fragmenten zertrümmern. Für die Feinfragmente wird dabei eine Partikelgröße erhalten, die ein meistens problemloses Absaugen durch einen axialen Hohlraum der Sonde hindurch bis hin zu ihrem proximalen Ende erlaubt, an welchem ein durch den Ultraschallwandler hindurchgeführter Sauganschluß zur Wirkung gebracht ist. Die Steinzertrümmerung mittels solcher Ultraschall-Lithotripter ist jedoch relativ zeitaufwändig, da sich mit dem distalen Ende der Sonotrode bei den für eine gewebeschonende Steinbearbeitung üblichen Ultraschallfrequenzen von etwa 20 bis 25 kHz und Amplituden der Sondenspitze bis etwa 50 µm die Steinzertrümmerung nur reichlich langsam fortführen läßt. Erschwerungen ergeben sich dabei auch bei den härteren Körpersteinen, die sich bei den Ultraschallfrequenzen und den Amplituden dieser Größenordnung nicht spontan abschaben lassen, sodaß ihre Bearbeitung entweder sehr lange dauert oder überhaupt nicht möglich ist.

Aus der EP 0 317 507 B1 ist ein Lithotripter bekannt, bei welchem das proximale Ende einer Metallsonde bzw. eines Wellenleiters mit einem pneumatisch angetriebenen Schlagteil beaufschlagt wird, um mit einer dadurch erhaltenen Stoßenergie eine die Metallsonde bis hin zu ihrem distalen Ende durchlaufende Stoß- bzw. Druckwelle zu erzeugen. Mit dieser Stoß- bzw. Druckwelle wird auf die Körpersteine eingewirkt, wobei die Körpersteine für eine vergleichbare Zertrümmerung mit der Sondenspitze in Berührung gebracht werden. Solche Stoßwellen-Lithotripter, die bei anderen Ausführungen auch einen elektrischen Antrieb des Schlagteils aufweisen können, ergeben allgemein einen relativ einfachen Geräteaufbau bei ebenfalls sehr guten Fragmentierungsleistungen auch an härteren Körpersteinen. Bei diesen Lithotriptern muß jedoch die Steinzertrümmerung bis hin zu einer absaugfähigen Partikelgröße immer noch relativ zeitaufwändig fortgeführt werden.

Der Erfindung liegt die Aufgabe zugrunde, eine Vorrichtung der eingangs genannten Art derart auszubilden, daß damit unter Berücksichtigung der Vor- und Nachteile dieser bekannten Verfahren eine flexiblere Steinzertrümmerung durchführbar ist.

Diese Aufgabe wird erfindungsgemäß gelöst mit einer Vorrichtung der durch den Anspruch 1 angegebenen Ausbildung, die mit den Merkmalen der weiteren Ansprüche eine vorteilhafte Gestaltungsmöglichkeit erfährt.

Bei der erfindungsgemäßen Vorrichtung ist somit die Möglichkeit geschaffen, eine mittels eines Endoskops durchgeführte Steinzertrümmerung entweder durch eine elektrische Ansteuerung des Ultraschallwandlers oder durch eine Einschaltung des reversiblen Antriebs für das Schlagteil einzuleiten. Dadurch wird entweder das distale Ende der Sonotrode durch den Ultraschallwandler zu periodischen Schwingungen angeregt, oder es wird das proximale Ende der Stoßsonde mit einer Stoßkraft beaufschlagt, um mit der dadurch erhaltenen Stoßenergie die Abgabe von Druckimpulsen an die Körpersteine durch das distale Ende der Stoßsonde zu erzeugen. Mit dieser alternativen Möglichkeit kann daher die Steinzertrümmerung sehr weitreichend optimiert werden. Mit der erfindungsgemäßen Ausbildung der Vorrichtung wird gleichzeitig der besondere Vorteil erhalten, daß das Überwechseln von der einen in die andere Möglichkeit einer Steinzertrümmerung auch während der Behandlung eines Patienten ohne eine zwingende Notwendigkeit für einen Austausch der Sonotrode gegen die Stoßsonde vorgenommen werden kann. In jedem Moment der Steinzertrümmerung kann deshalb auch entschieden werden, mit welcher der beiden Alternativen eine spontan abgangsfähige oder auch aus dem Körper direkt absaugfähige bzw. ausspülbare Partikelgröße erhalten werden kann. Daneben vereinfacht die erfindungsgemäße Vorrichtung auch die Entscheidung, welche der beiden Alternativen im Einzelfall auch unter dem Gesichtspunkt zweckmäßiger ausgewählt wird, schädigende Nebeneffekte auf das biologische Gewebe in der unmittelbaren Nähe der zu zertrümmernden Körpersteine zu vermeiden, wobei eine solche Entscheidung wegen der zuvor unbekannten Härte sowie der Zusammensetzung und auch der Größe der Körpersteine häufig erst während des chirurgischen Eingriffs zu treffen ist.

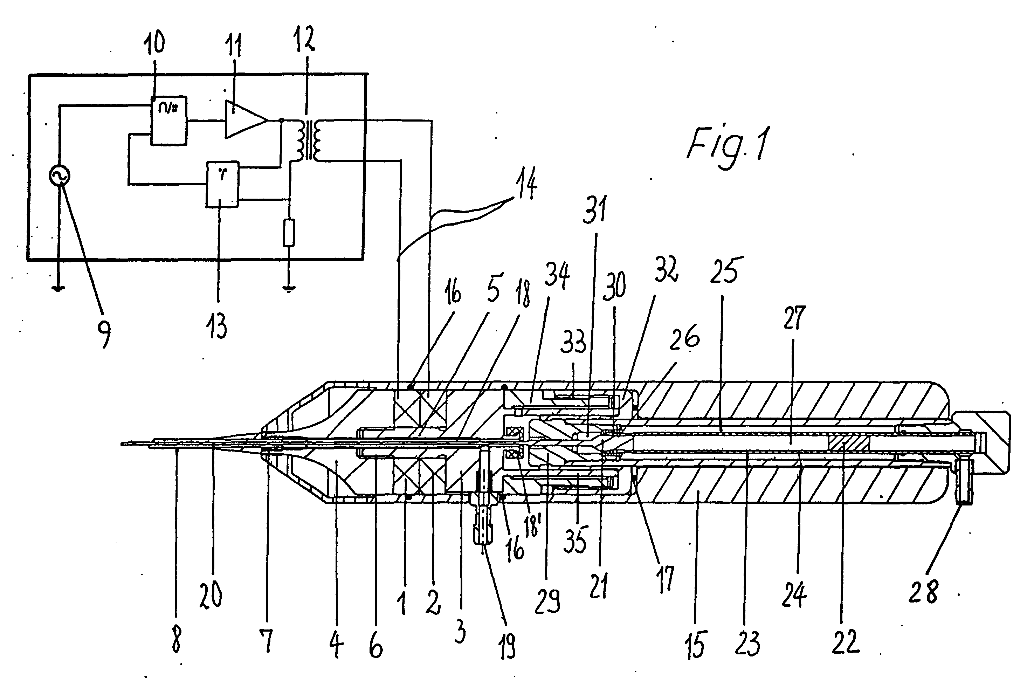

Ein Ausführungsbeispiel der erfindungsgemäßen Vorrichtung ist in der Zeichnung schematisch dargestellt und wird nachfolgend näher erläutert. Es zeigt
- Fig. 1: eine Schnittansicht der Vorrichtung zusammen mit einem Schaltbild der Schaltungsanordnung für den Ultraschallwandler und
- Fig. 2: eine Schnittansicht in vergrößertem Maßstab eine Einzelheit der Vorrichtung in der Umgebung des Sondenkopfes der Stoßsonde.

Die in der Zeichnung dargestellte Vorrichtung zum Entfernen von Körpersteinen unter Verwendung eines intrakorporalen Lithotripters besteht auf der einen Seite aus einem elektrisch angesteuerten Ultraschallwandler, der als ein piezoelektrischer Wandler mit zwei piezokeramischen Scheiben 1, 2 in einer Anordnung zwischen einem Reflektor 3 und einem Horn 4 ausgebildet ist. Die beiden piezokeramischen Scheiben 1, 2 sind durch einen axialen Fortsatz 5 des Reflektors 3 zentriert, der an seinem Ende mit einem Schraubansatz 6 versehen ist, auf welchen das mit einem komplementären Innengewinde versehene Horn 4 für ein gegenseitiges Verspannen der einzelnen Teile des Ultraschallwandlers aufschraubbar ist.

Das Horn 4 des Ultraschallwandlers ist mit einer sich in axialer Richtung etwa auf den Querschnitt eines Schraubansatzes 7 einer hohlen Metallsonde bzw. Sonotrode 8 verjüngenden Hüllfläche in der Ausbildung einer Exponentialkurve versehen. Anstelle der Exponentialform kann für die Hüllfläche des Horns 4 auch eine konische oder eine stufenförmige Ausbildung vorgesehen sein. Um optimale Ergebnisse bei der Steinzertrümmerung zu erhalten, sollten das Horn 4 und die hohle Metallsonde bzw. Sonotrode 8 zweckmäßig aus einem Material mit einer im wesentlichen gleichen akustischen Impedanz bestehen. Bevorzugte Materialien sind Edelstahl oder Titan.

Für die elektrische Ansteuerung des Ultraschallwandlers ist eine herkömmlich ausgebildete Schaltungsanordnung vorgesehen. Die Schlatungsanordnung ist mit einem spannungsgesteuerten Oszillator 9 gebildet ist, dessen Ausgangssignal über einen U/F-Wandler 10 und einen nachgeschalteten Ausgangsverstärker 11 an einen Impedanz-Transformator 12 angeliefert wird. An den Transformator 12 ist weiterhin ein mit dem U/F-Wandler 10 verbundener Phasenkomparator 13 angeschlossen, der die Phasen der Ausgangsspannung und des Ausgangsstroms miteinander vergleicht und eine Regelspannung zur Steuerung des Oszillators 9 erzeugt. Der Impedanz-Transformator 12 ist über Anschlußleitungen 14 an die beiden piezokeramischen Scheiben 1 und 2 des Ultraschallwandlers angeschlossen. Mit diesem Anschluß kann daher die hohle Metallsonde bzw. Sonotrode 8 zu periodischen, longitudinalen Schwingungen angeregt werden.

Die mit den beiden piezokeramischen Scheiben 1, 2, dem Reflektor 3 und dem Horn 4 gebildete Anordnung des Ultraschallwandlers ist gegen ein umgebendes Gehäuse 15 durch elastische Stützmittel 16 und 17 abgestützt. Ein rückwärtiger Teil des Gehäuses 15 ist als ein Handgriff zum Halten der Vorrichtung entsprechend massiver ausgebildet ist als der vordere Gehäuseteil, der die Einzelteile des Ultraschallwandlers aufnimmt. Die Einzelteile des Ultraschallwandlers, also die beiden piezokeramischen Scheiben 1, 2, der Reflektor 3 und das Horn 4, welche durch den axialen Fortsatz 5 des Reflektors 3 gegeneinander verspannt sind, weisen eine axiale Durchgangsbohrung 18 auf, die mit dem Hohlraum der Sonotrode 8 axial fluchtet und mit einem Sauganschluß 19 verbunden ist, über welchen die bei der Steinzertrümmerung mittels der Sonotrode 8 anfallenden Partikel aus dem Körper abgesaugt bzw. ausgespült werden können. Die axiale Durchgangsbohrung 18 ist gleichzeitig so groß bemessen, daß sie auch eine Stoßsonde 20 aufnehmen kann. Die Länge dieser Stoßsonde 20 ist so bemessen, daß ihr distales Ende etwas über das distale Ende der Sonotrode 8 vorsteht, wenn die Steinzertrümmerung nicht mit einer elektrischen Ansteuerung des Ultraschallwandlers für eine periodische Schwingunsanregung der Sonotrode 8 durchgeführt wird, sondern mit dieser Stoßsonde 20 durchgeführt werden soll. Die Stoßsonde 20 kann flexibel oder starr ausgebildet sein und durchdringt für ihre axiale Anordnung in der hohlen Sonotrode 8 eine kappenförmige Dichtungsmanschette 18', mit welcher die Durchgangsbohrung 18 des Ultraschallwandlers an der Rückseite des Reflektors 3 abgedichtet ist.

Die Stoßsonde 20 bildet ein Arbeitselement eines Funktionsteils der Vorrichtung, welcher alternativ zu dem elektrisch angesteuerten Ultraschallwandler für die Steinzertrümmerung benutzt werden kann und im wesentlichen gleich ausgeführt ist wie die bekannten intrakorporalen Stoßwellen-Lithotripter gemäß der EP 0 317 507 B1. Die Stoßsonde 20 ist daher an ihrem proximalen Ende mit einem Massekörper 21 versehen, auf welchen durch ein reversibel angetriebenes Schlagteil 22 eine Stoßkraft ausgeübt werden kann. Als Folge der auf den Massekörper 21 übertragenen Stoßenergie kommt es dabei zu einer Ausbildung von Stoß- bzw. Druckwellen in der Stoßsonde 20, die dann als Druckimpulse über das distale Sondenende in einen zu zertrümmernden Körperstein eingeleitet werden. Der reversible Antrieb des Schlagteils 22 ist bevorzugt pneumatisch ausgeführt, sodaß für eine ergänzende Beschreibung von Einzelheiten dieses pneumatischen Antriebs auf die vorerwähnte EP 0 317 507 B1 verwiesen werden kann. Alternativ kann das Schlagteil 22 aber auch hydraulisch oder elektromagnetisch angetrieben sein, wobei neben einer direkten Schlageinwirkung auf den Massekörper 21 auch eine indirekte Ausübung der Stoßkraft angedacht werden kann.

Das Schlagteil 22 ist in einer Führungshülse 23 aufgenommen, die mit der axialen Durchgangsbohrung 18 des Ultraschallwandlers und mit dem Hohlraum der Sonotrode 8 axial fluchtet. In die Führungshülse 23 kann daher das über den Reflektor 3 des Ultraschallwandlers axial nach rückwärts vorstehende und mit dem Massekörper 21 versehene proximale Ende der Stoßsonde 20 einfassen. Die Führungshülse 23 ist von einem koaxial angeordneten Mantelrohr 24 umgeben, wobei zwischen dem Führungsrohr und dem Mantelrohr eine Reversierkammer 25 des pneumatischen Antriebs für das Schlagteil 22 ausgebildet ist. Die Reversierkammer 25 ist an einem Fenster 26 mit einer mit der Führungshülse 23 bereitgestellten Druckkammer 27 verbunden. Die Druckkammer 27 ist durch einen mit dem Mantelrohr 25 verschraubten Druckanschluß 28 verschlossen.

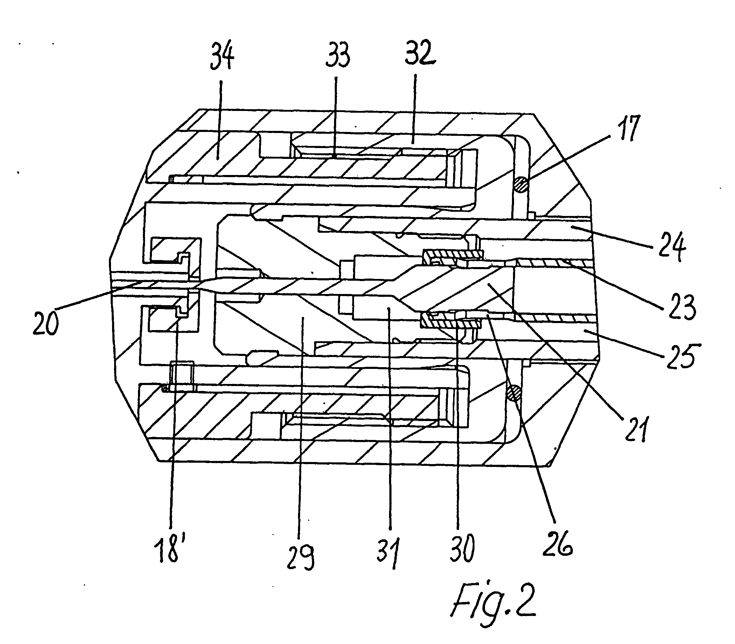

Wie insbesondere aus der Einzeldarstellung der Fig. 2 näher ersichtlich ist, ist in das Mantelrohr 24 in der Nähe des proximalen Endes der Stoßsonde 20 ein axial gebohrter Einsatzkörper 29 eingesetzt, der an dem Fenster 26 durch eine Dichtung 30 gegen die Führungshülse 23 abgedichtet ist. Gegen diesen Einsatzkörper 29 ist der Massekörper 21 des proximalen Endes der Stoßsonde 20 durch ein Dämpfungselement 31 abgestützt, mit welchem somit die auf den Massekörper 21 durch das Schlagteil 22 ausgeübten Stoßkräfte gedämpft und vergleichmäßigt werden.

Der Einsatzkörper 29 wird durch eine Schraubkappe 32 gehalten, die an einem Schraubansatz 33 mit einer Zentrierhülse 34 verschraubt ist. Durch die Zentrierhülse 34 wird die Führungshülse 23 unter Mitwirkung des Einsatzkörpers 29 und der Schraubkappe 32 in einem rückwärtigen rohrförmigen Ansatz 35 des Reflektors 3 zentriert. Diese Zentrierung ist mit den elastischen Stützmitteln 16 und 17 an dem Gehäuse 15 abgestützt.

Anstelle der hier beschriebenen Ausbildung des Massekörpers 21 mit einem vergrößerten Kopfteil der Stoßsonde 20 kann für den Massekörper auch eine von der Stoßsonde getrennte Ausbildung vorgesehen sein mit einer Anordnung wie beschrieben in der DE 196 18 972 A1, um damit die dort beschriebenen Vorteile der Wirkung eines solchen getrennten Massekörpers gleichzeitig als ein Dichtungsorgan und als ein Übertragungsorgan für die Stoßkräfte des Schlagteils auch für die erfindungsgemäße Vorrichtung zu nutzen.

Die elektrische Ansteuerung des Ultraschallwandlers kann andererseits noch dadurch optimiert werden, daß dafür eine Umschaltmöglichkeit zwischen einer periodischen Schwingungsanregung und einer impulsförmigen Schwingungsanregung der Sonotrode vorgesehen wird, die bsp. zu realisieren ist mit einer noch zusätzlich vorgesehenen Schaltungsanordnung mit einer Spannungsquelle und mit einem Kondensator, die dabei in eine Anschlußverbindung mit dem Ultraschallwandler umschaltbar wäre.

Die Vorrichtung ist zweckmäßig so ausgeführt, daß die als ein Stoßwellen-Lithotripter einsetzbaren Funktionsteile als eine gemeinsame Baugruppe von der Vorrichtung vollständig abgenommen werden können, sodaß nur noch die Funktionsteile, welche dem Ultraschallwandler zugeordnet sind, bei der Vorrichtung verbleiben. Die Vorrichtung kann dann also wie ein herkömmlicher Ultraschall-Lithotripter betrieben werden, unabhängig von der Möglichkeit eines dualen Betriebes der Vorrichtung auch als ein herkömmlicher Stoßwellen-Lithotripter.

## Patentansprüche

1. Vorrichtung zum Entfernen von Körpersteinen mit einem intrakorporalen Lithotripter, welcher eine hohle Metallsonde bzw. Sonotrode aufweist, die für eine mit ihrem distalen Ende veranlaßte Steinzertrümmerung in den Arbeitskanal eines Endoskops eingesetzt und durch einen elektrisch angesteuerten Ultraschallwandler zu longitudinalen Schwingungen angeregt wird, wobei der Ultraschallwandler mit wenigstens einer piezokeramischen Scheibe gebildet ist, die innerhalb eines umgebenden Gehäuses zwischen einem Reflektor und einem die Sonotrode tragenden Horn verspannt ist
**dadurch gekennzeichnet, daß** das Gehäuse (15) eine mit der hohlen Sonotrode (8) axial fluchtende Führungshülse (23) für ein reversibel angetriebenes Schlagteil (22) aufnimmt, welches zur Ausübung einer Stoßkraft gegen einen Massekörper (21) an dem über den Reflektor (3) des Ultraschallwandlers axial nach rückwärts in die Führungshülse (23) vorstehenden proximalen Ende einer Stoßsonde (20) angeordnet ist, die zu ihrem distalen Ende hin durch eine axiale Durchgangsbohrung (18) des Ultraschallwandlers hindurchgeführt und in dem Hohlraum der Sonotrode (8) aufgenommen ist.

2. Vorrichtung nach Anspruch 1, **dadurch gekennzeichnet, daß** die Stoßsonde (20) relativ zu der hohlen Sonotrode (8) axial verstellbar ist.

3. Vorrichtung nach Anspruch 1 oder 2, **dadurch gekennzeichnet, daß** die axiale Durchgangsbohrung (18) des Ultraschallwandlers über eine Querbohrung mit einem Sauganschluß (19) verbunden ist.

4. Vorrichtung nach einem der Ansprüche 1 bis 3, **dadurch gekennzeichnet, daß** die Durchgangsbohrung (18) durch eine an der Rückseite des Reflektors (3) angeordnete Dichtungskappe (18') abgedichtet ist.

5. Vorrichtung nach einem der Ansprüche 1 bis 4, **dadurch gekennzeichnet, daß** die mit der wenigstens einen piezokeramischen Scheibe (1,2), dem Reflektor (3) und dem Horn (4) gebildete Anordnung des Ultraschallwandlers durch eine zu der axialen Durchgangsbohrung (18) koaxiale Spannschraube zusammengehalten sind.

6. Vorrichtung nach Anspruch 5, **dadurch gekennzeichnet, daß** die Spannschraube mit einem axialen Fortsatz (5) des Reflektors (3) ausgebildet ist, auf welchem die wenigstens eine piezokeramische Scheibe (1,2) zentriert und welcher an einem Schraubansatz (6) mit dem Horn (4) verschraubt ist.

7. Vorrichtung nach einem der Ansprüche 1 bis 6, **dadurch gekennzeichnet, daß** die mit der wengistens einen piezokeramischen Scheibe (1,2), dem Reflektor (3) und dem Horn (4) gebildete Anordnung des Ultraschallwandlers durch elastische Stützmittel (16, 17) gegen das umgebende Gehäuse (15) abgestützt ist.

8. Vorrichtung nach einem der Ansprüche 1 bis 7, **dadurch gekennzeichnet, daß** die Führungshülse (23) für das reversibel angetriebene Schlagteil (22) in einem rückwärtigen rohrförmigen Ansatz (35) des Reflektors (3) des Ultraschallwandlers zentriert ist.

9. Vorrichtung nach einem der Ansprüche 1 bis 8, **dadurch gekennzeichnet, daß** die Führungshülse (23) über einen massiven, axial gebohrten Einsatzkörper (29) in dem rohrförmigen Ansatz (35) des Reflektors (3) aufgenommen ist.

10. Vorrichtung nach Anspruch 9, **dadurch gekennzeichnet, daß** der Einsatzkörper (29) ein Dämpfungselement (31) zur Abstützung des Massekörpers (21) an dem proximalen Ende der Stoßsonde (20) aufweist.

11. Vorrichtung nach einem der Ansprüche 8 bis 10, **dadurch gekennzeichnet, daß** der rohrförmige Ansatz (35) des Reflektors (3) in einer durch das umgebende Gehäuse (15) zentrierten Zentrierhülse (34) aufgenommen ist.

12. Vorrichtung nach einem der Ansprüche 8 bis 11, **dadurch gekennzeichnet, daß** der Einsatzkörper (29) mit der Zentrierhülse (34) durch eine Schraubkappe (32) verbunden ist, die an dem umgebenden Gehäuse (15) durch ein elastisches Stützmittel (16, 17) abgestützt ist.

13. Vorrichtung nach einem der Ansprüche 1 bis 12, **dadurch gekennzeichnet, daß** für einen pneumatischen Antrieb des Schlagteils (22) die Führungshülse (23) mit einem Druckluftanschluß (28) versehen und von einem koaxial angeordneten Mantelrohr (24) mit Abstand umgeben ist zur Ausbildung einer mit einer Druckkammer (27) der Führungshülse (23) über ein Fenster (26) verbundenen Reversierkammer (25) des pneumatischen Antriebs, wobei die Reversierkammer (25) durch den in das Mantelrohr (24) als ein Verschlußkörper eingesetzten und gegen die Führungshülse (23) abgedichtet festgelegten Einsatzkörper (29) verschlossen ist.

14. Vorrichtung nach einem der Ansprüche 1 bis 13, **dadurch gekennzeichnet, daß** die zu der Stoßsonde (20) zugehörigen Funktionsteile (21, 22, 23, 24, 28, 29, 30) als eine gemeinsame Baugruppe ausgeführt sind, die aus dem umgebenden Gehäuse (15) für einen Verbleib nur der Funktionsteile (1, 2, 3, 4, 5, 8), des Ultraschallwandlers entnommen werden kann.

## Claims

1. Device for removal of calculi by means of an intracorporeal lithotripter which comprises a hollow metallic probe or a sonotrode respectively which is inserted into the operating passage of an endoscope for a fragmentation of calculi via its distal end and which is excited by an electrically controlled ultrasonic transducer for generating longitudinal oscillations whereby said ultrasonic transducer is composed of at least one piezoceramic disc which within a surrounding casing is prestressed between a reflector and a horn that carries said sonotrode,
**characterised in that** said casing (15) accommodates a guide bush (23) in axial alignment with said hollow sonotrode (8) for a reversibly driven impact member (22) which is arranged for generating an impact force against a mass body (21) on the proximal end of an impact probe (20) which proximal end projects axially backwards over the reflector (3) of the ultrasonic transducer and into the guide bush (23), said impact probe being passed towards its distal end through an axial passage bore (18) of said ultrasonic transducer and being accommodated in the hollow of said sonotrode (8).

2. The device according to claim 1, **characterised in that** said impact probe (20) is axially adjustable relative to said hollow sonotrode (8).

3. The device according to claim 1 or 2, **characterised in that** said axial passage bore (18) of the ultrasonic transducer is connected via a transverse bore with a suction duct (19).

4. The device according to any of the claims 1 to 3, **characterised in that** said passage bore (18) is sealed by means of a sealing cap (18') which is arranged at the rear side of said reflector (3).

5. The device according to any of the claims 1 to 4, **characterised in that** the arrangement of the ultrasonic transducer which is horned by means of said at least one piezoceramic disc (1, 2), said reflector (3) and said horn (4) being held together by means of a straining screw coaxial with said axial passage bore (18).

6. The device according to claim 5 **characterised in that** said straining screw is horned by an axial extension (5) of said reflector (3) whereby at least said one piezoceramic disc (1, 2) is centered on said extension which at a thread portion (6) is screwed together with said horn (4).

7. The device according to any of the claims 1 to 6, **characterised in that** the arrangement of the ultrasonic transducer which is horned with said at least one piezoceramic disc (1, 2), said reflector (3) and said horn (4) is supported on said surrounding casing (15) by means of elastic supporting means (16, 17).

8. The device according to any of the claims 1 to 7, **characterised in that** said guide bush (23) for said reversibly driven impact member (22) is centered in a rear tubular extension (35) of said reflector (3) of the ultrasonic transducer.

9. The device according to any of the claims 1 to 8, **characterised in that** said guide bush (23) is accommodated by means of a massive and axially bored insert body (29) in said tubular extension (35) of said reflector (3).

10. The device according to claim 9, **characterised in that** said insert body (29) comprises a damping element (31) for supporting said mass body (21) on the proximal end of the impact probe (20).

11. The device according to any of the claims 8 to 10, **characterised in that** said tubular extension (35) of the reflector (3) is accommodated in a centering bush (34) which is centered by the surrounding casing (15).

12. The device according to any of the claims 8 to 11, **characterised in that** said insert body (29) is connected with said centering bush (34) by means of a screw cap (32) which is supported on the surrounding casing (15) by means of elastic supporting means (16, 17).

13. The device according to any of the claims 1 to 12, characterised that for a pneumatic drive of said impact member (22) said guide bush (23) is provided with a connection (28) for compressed air and is surrounded by a coaxially arranged outer tube (24) with an interspace for providing a reversing chamber (25) of the pneumatic drive which is connected with a pressure chamber (27) of said guide bush (23) via a window (26) whereby said reversing chamber (25) is closed by said insert body (29) which is inserted into said outer tube (24) as a closure member which is sealed against said guide bush (23).

14. The device according to any of the claims 1 to 13, **characterised in that** the functional components (21, 22, 23, 24, 28, 29, 30) belonging to said impact probe (20) are all designed as a common subassembly which may be removed from the surrounding casing (15) so that the functional components (1, 2, 3, 4, 5, 8) of the ultrasonic transducer will remain in situ.

## Revendications

1. Dispositif à sortir des calculs moyennant un lithotriteur intracorporel, qui comprend une sonde métallique creuse ou respectivement sonotrode, qui est introduite dans le canal de travail d'un endoscope pour une opération de broyage des calculs, qui est induite par son extrémité distale, et qui est excitée par un transducteur d'ultrasons de manière à subir des vibrations longitudinales, ledit transducteur d'ultrasons étant configuré à au moins un disque piézoélectrique, qui est serré au-dedans d'un boîtier l'entourant entre un réflecteur et une corne qui porte ladite sonotrode,
**caractérisé en ce que** ledit boîtier (15) reçoit une douille de guidage (23) en alignement axial avec ladite sonotrode creuse (8) pour un élément de chasse (22) entraîné pour un mouvement va-et-vient, qui, pour la réalisation d'un effort de chasse contre un corps de masse (21), est disposé à l'extrémité proximale d'une sonde de chasse (20), qui se trouve en saillie dudit réflecteur (3) dudit transducteur d'ultrasons en sens axial vers l'arrière dans ladite douille de guidage (23), à ladite sonde de chasse (20) étant passée à travers d'un alésage de passage (18) dudit transducteur d'ultrasons vers son extrémité distale et étant reçue dans la cavité de ladite sonotrode (8).

2. Dispositif selon la revendication 1, **caractérisé en ce que** ladite sonde de chasse (20) est axialement ajustable relativement à ladite sonotrode creuse (8).

3. Dispositif selon la revendication 1 ou 2, **caractérisé en ce que** ledit alésage de passage (18) dudit transducteur d'ultrasons est raccordé via un alésage transversal à un raccord d'aspiration (19).

4. Dispositif selon une quelconque des revendications 1 à 3, **caractérisé en ce que** ledit alésage de passage (18) est rendu étanche moyennant un bouchon d'étanchéité (18') disposé du côté arrière dudit réflecteur (3).

5. Dispositif selon une quelconque des revendications 1 à 4, **caractérisé en ce que** l'arrangement dudit transducteur d'ultrasons, qui est constitué par ledit au moins un disque piezo céramique (1, 2), ledit réflecteur (3) et ladite corne (4), est serré ensemble moyennant un écrou de serrage coaxial avec ledit alésage axial de passage (18).

6. Dispositif selon la revendication 5, **caractérisé en ce que** ledit écrou de serrage est muni d'un prolongement axial (5) dudit réflecteur (3), sur lequel ledit au moins un disque (1, 2) est centré et qui est vissé à ladite corne (4) à un bout de vissage (6).

7. Dispositif selon une quelconque des revendications 1 à 6, **caractérisé en ce que** l'arrangement dudit transducteur d'ultrasons, qui est constitué par ledit au moins un disque piezo céramique (1, 2), ledit réflecteur (3) et ladite corne (4), est appuyé sur le boîtier l'entourant (15) moyennant des moyens élastiques d'appui (16, 17).

8. Dispositif selon une quelconque des revendications 1 à 7, **caractérisé en ce que** ladite douille de guidage (23) pour ledit élément de chasse entraîné pour un mouvement va-et-vient est centrée dans un bout tubulaire (35) du côté arrière dudit réflecteur (3) du transducteur d'ultrasons.

9. Dispositif selon une quelconque des revendications 1 à 8, **caractérisé en ce que** ladite douille de guidage (23) est reçue dans ledit bout tubulaire (35) dudit réflecteur via un corps d'insert (29) solide à alésage axial.

10. Dispositif selon la revendication 9, **caractérisé en ce que** ledit corps d'insert (29) comprend un élément amortisseur (31) à appuyer ledit corps de masse (21) à l'extrémité proximale de ladite sonde de chasse (20).

11. Dispositif selon une quelconque des revendications 8 à 10, **caractérisé en ce que** ledit bout tubulaire (35) dudit réflecteur est reçu dans une douille de centrage (34) centrée par le boîtier (15) l'entourant.

12. Dispositif selon une quelconque des revendications 8 à 11, **caractérisé en ce que** ledit corps d'insert (29) est relié à ladite douille de centrage (34) moyennant un bouchon fileté (32), qui s'appuie au boîtier (15) l'entourant moyennant un moyen élastique d'appui (16, 17).

13. Dispositif selon une quelconque des revendications 1 à 12, **caractérisé en ce que** ladite douille de guidage (23) est pourvue d'un raccord de l'air comprimé (28) pour l'entraînement pneumatique dudit élément de chasse (22) et est entourée par un tube enveloppe (24) disposé, à un écart, en position coaxiale afin de former une chambre à va-et-vient (25) du système d'entraînement pneumatique, qui est reliée à une chambre de pression (27) de ladite douille de guidage (23) via une fenêtre (26) et qui est fermée par ledit corps d'insert (29) inséré dans ledit tube enveloppe (24) comme corps obturateur, qui est fixé, de façon étanche, en appui contre ladite douille de guidage (23).

14. Dispositif selon une quelconque des revendications 1 à 13, **caractérisé en ce que** les éléments fonctionnels (21, 22, 23, 24, 28, 29, 30), qui appartiennent à ladite sonde de chasse (20), sont conçu sous forme d'un module commun qui est apte à être enlevé en dehors dudit boîtier (15) entourant de manière, que seulement les éléments fonctionnels (1, 2, 3, 4, 5, 8) dudit transducteur d'ultrasons y restent.
